# EUROPEAN PATENT SPECIFICATION

(11) **EP 2 942 033 B1**
(45) Date of publication and mention of the grant of the patent: **17.08.2016**
(21) Application number: 14171954.2
(22) Date of filing: 11.06.2014
(51) Int. Cl.: A61F 2/00

(54) **Artificial urinary sphincter with iris diaphragm and method of providing urinary continence**
Künstlicher Blasenschließmuskel mit Irisdiaphragma und Verfahren zur Bereitstellung von Harnkontinenz
Sphincter urinaire artificiel avec diaphragme iris et procédé de fourniture de continence urinaire

(30) Priority: 06.05.2014 US 201414270367
(43) Date of publication of application: 11.11.2015
(73) Proprietor: Coloplast A/S, 3050 Humlebaek (DK)
(72) Inventor: Deitch, Sarah J., Minneapolis, MN 55417 (US)

(56) References cited:
- EP-A2- 1 586 283
- US-A- 4 118 805
- US-A- 4 551 862
- US-A- 4 705 518
- US-A1- 2002 111 530

## Description

### Background

Urinary incontinence affects about 200 million people worldwide and about 25 million people in the US. Urinary incontinence is generally more prevalent in women than in men.

Urinary incontinence in women can be associated with a prolapse of one or more pelvic organs, which can arise from a weakness in the tissues / muscle of the pelvic floor. Urinary incontinence in men can arise after surgical treatment of the prostate glade, which treatment can include removal or weakening of the prostatic sphincter associated with the urinary urethra.

One treatment for urinary incontinence includes placing an artificial sphincter around a portion of the urethra. The artificial sphincter has a closed position that selectively prevents the flow of urine through the urethra, thus providing the user with a comfortable, continent state. The artificial sphincter can be activated to an open position by the user, which opens the urethra and allows the user to selectively pass urine.

US4705518 discloses an artificial sphincter apparatus including a pair of parallel, coaxially mounted rings with at least one ring rotatable relative to the other.

Surgeons and patients would welcome advances in the treatment of urinary incontinence.

### Summary

One aspect provides an artificial urinary sphincter (AUS) according to the appended claims.

Also disclosed is an exemplary method of controlling urinary voiding in a male patient suffering from incontinence. The method includes providing an AUS having a casing sized for implantation between bulbous spongiosis muscle and perineal skin, a coaptation valve provided with an iris diaphragm and a movable element and engaging the AUS with the urethra of the patient. The method further includes moving the movable element in a first direction to increase a diameter of the iris diaphragm and permit flow of urine through the urethra. The method further includes moving the movable element in a second, opposite direction to reduce the diameter of the iris diaphragm to a coapting position and prevent the flow of urine through the urethra.

Also disclosed is an exemplary method of providing urinary voiding control in a male patient suffering from incontinence. The method includes providing an AUS having a casing sized for implantation between bulbous spongiosis muscle and perineal skin, a coaptation valve provided with an iris diaphragm and a movable element. The method further includes making a perineal incision in the patient and dissecting tissue to expose a section of the patient's urethra. The method further includes configuring the AUS to be engageable with the urethra and positioning the movable element such that it is in position for being palpated through the perineal skin surface of the patient. The method further includes closing of the perineal incision in the patient.

One aspect provides a kit of parts for providing urinary continence including the AUS according to the first aspect.

### Brief Description of the Drawings

The accompanying drawings are included to provide a further understanding of embodiments and are incorporated in and constitute a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Figure 1 is a schematic perspective view of one embodiment of an artificial urinary sphincter (AUS) located at the bulbar urethra in the urogenital region of a male patient.
Figure 2 is a top view of one embodiment of an artificial urinary sphincter.
Figure 3 is a perspective exploded view of one embodiment of an artificial urinary sphincter.
Figure 4A is a top view of one embodiment of an artificial urinary sphincter with an iris diaphragm adjusted to a first diameter.
Figure 4B is a top view of the embodiment of Figure 4A with the iris diaphragm adjusted to a second diameter.
Figure 4C is a top view of the embodiment of Figure 4A with some elements not illustrated.
Figure 5A is a top view of one embodiment including a biasing member in a first configuration.
Figure 5B is a top view of one embodiment including a biasing member in a second configuration.
Figure 5C is an enlarged sectional view of one embodiment including a biasing member.
Figure 6A is an enlarged sectional perspective view of a portion of one embodiment of an AUS including a movable element accommodated in a slit.
Figure 6B is an enlarged sectional perspective view of a portion of another embodiment of an AUS including a movable element accommodated in a slit.
Figure 7 is a schematic view of one embodiment of an artificial urinary sphincter located in tissue around a urethra of a male patient.
Figure 8 is a top view of one embodiment of a kit of parts for providing urinary continence.
Figure 9 is a block diagram illustrating one example of a method of providing urinary control in an incontinent male patient.
Figure 10 is a block diagram illustrating one example of a method of controlling urinary voiding in an incontinent male patient.

### Detailed Description

In the following Detailed Description, reference is made to the accompanying drawings, which form a part hereof, and in which is shown by way of illustration specific embodiments in which the invention may be practiced. In this regard, directional terminology, such as "top," "bottom," "front," "back," "leading," "trailing," etc., is used with reference to the orientation of the Figure(s) being described. Because components of embodiments can be positioned in a number of different orientations, the directional terminology is used for purposes of illustration and is in no way limiting. It is to be understood that other embodiments may be utilized and structural or logical changes may be made without departing from the scope of the present invention. The following detailed description, therefore, is not to be taken in a limiting sense, and the scope of the present invention is defined by the appended claims.

It is to be understood that the features of the various exemplary embodiments described herein may be combined with each other, unless specifically noted otherwise.

Tissue includes soft tissue, which includes dermal tissue, sub-dermal tissue, ligaments, tendons, or membranes. As employed in this specification, the term "tissue" does not include bone.

In this specification, end means endmost and end portion means that segment that is adjacent to and extends from the end.

In this specification, generally annular means that an element overall is ring shaped, but not necessarily a circle. Also, while the element can be continuous, the element is not necessarily continuous and may include an open segment or gap.

Embodiments provide an artificial urinary sphincter (AUS) that is implantable in the perineal region of a patient suffering from incontinence. The AUS is located around the urethra and includes a casing, a coaptation valve including an iris diaphragm and a movable element. The iris diaphragm has an adjustable diameter. When the AUS is implanted, the movable element is adjustable through the perineal skin surface to change the diameter of the iris diaphragm. To empty the bladder, the iris diaphragm is adjusted to a relatively large diameter to move it away from the urethra and to allow urine flow through the urethra. To stop or prevent urine from flowing, the iris diaphragm is adjustable to have a relatively small diameter sized to make the iris diaphragm engage with and coapt the urethra.

Embodiments provide an AUS having fewer parts as there are no reservoirs, pumps and connections to be implanted, and it is easily adjustable by the patient herself / himself by moving the movable element through the perineal skin. Embodiments provide an AUS capable of maintaining pressure on the urethra in its coapting position and that is less susceptible to bodily movements of the patient due to the fewer interconnected parts which reduces involuntarily urine leakages.

The AUS described in this patent application is suited for use in male and female patients. The AUS is suitably implanted around the bulbar urethra of the male patient or around the junction of the urethra and the bladder neck of the female patient. The following description is generally directed to an AUS useful with a male patient, although it is to be understood that a similar such AUS may be adapted for use with a female patient.

Embodiments provide a method of providing urinary control in an incontinent male patient by implanting an AUS and positioning it around the urethra and placing the movable element so that it can be moved through the perineal skin. Implanting of the AUS by the presented method is improved since the AUS includes fewer parts than most sphincter systems presently in the market.

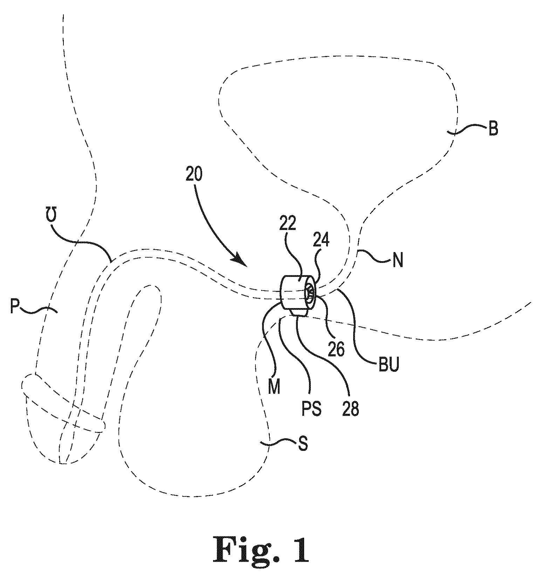

Figure 1 is a perspective view of one embodiment of an artificial urinary sphincter 20 (AUS 20) located around the bulbar urethra in the urogenital region of a male patient. The AUS 20 is placed around or in bulbous spongiosis muscle M which forms part of the tissue surrounding the bulbar urethra BU to locate the AUS 20 immediately downstream of bladder neck N. The position of the AUS 20 is inferior the perineal skin surface PS of the patient allowing the patient to palpate and move movable member 28 associated with casing 22 through the perineal skin posterior to scrotum S. Coaptation valve 24 of AUS 20 includes iris diaphragm 26 that is adjustable to coapt the bulbar urethra BU and prevent urine to flow from the bladder B through the bulbar urethra BU and penile urethra U of penis P and provide continence in the patient. Adjusting the AUS 20 to an open position where it does not coapt the bulbar urethra allows flow of urine. In other words, the AUS 20 provides continence in the patient by coapting the urethra and further provides the patient with the option to selectively control voiding of the bladder.

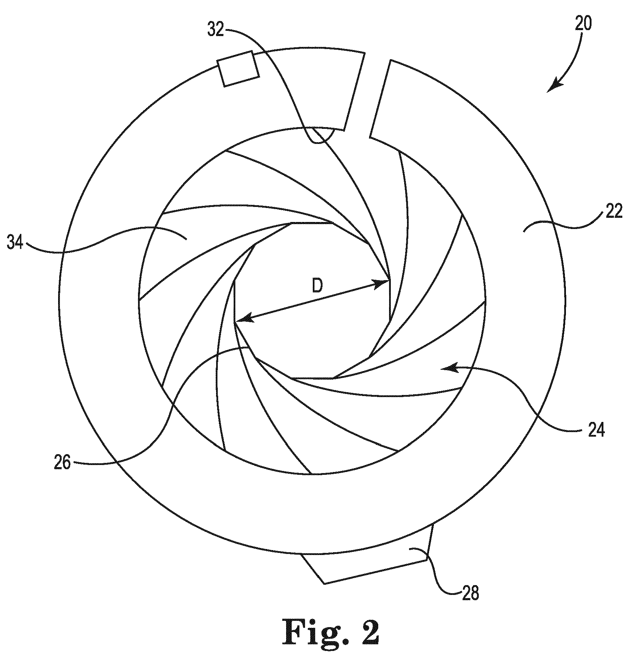

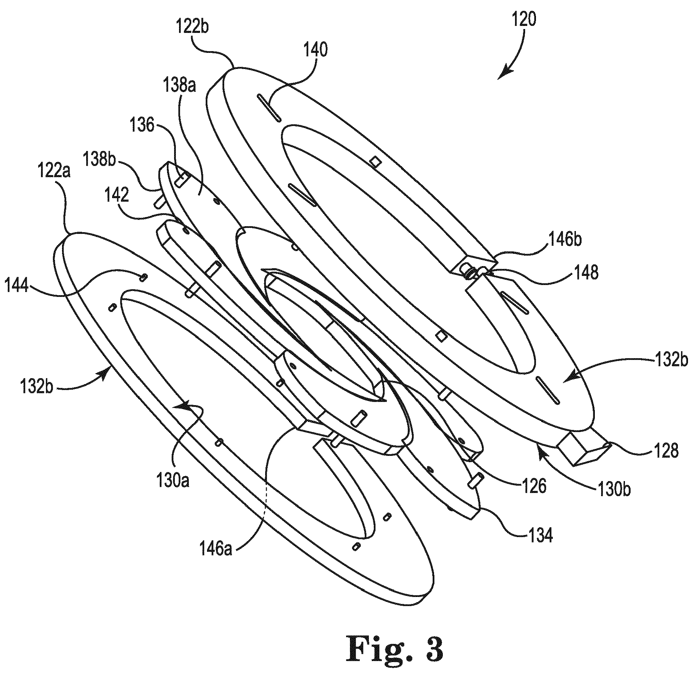

Figure 2 is a top view of one embodiment of an artificial urinary sphincter 20 (AUS 20). AUS 20 includes casing 22 and a coaptation valve 24 attachable to an interior surface 130 (Figure 3) of the casing 22 opposite of an exterior surface 32 of the casing 22. The coaptation valve 24 includes iris diaphragm 26 having an adjustable diameter D. The iris diaphragm 26 is movable to a coapting position wherein a patient's urethra (not shown) is encompassed and engaged by the iris diaphragm 26. In the coapting position, urine flow through the urethra is prevented.

The casing 22 is sized for implantation between bulbous spongiosis muscle and perineal skin and a movable element 28 is associated with the casing 22. In one embodiment, the casing 22 includes a set of opposed walls spaced apart to provide an accommodation space for the coaptation valve 24. In one embodiment, the casing includes a connection wall connecting the set of opposed walls. In one embodiment, the connection wall includes a trace in which the iris diaphragm 26 travels.

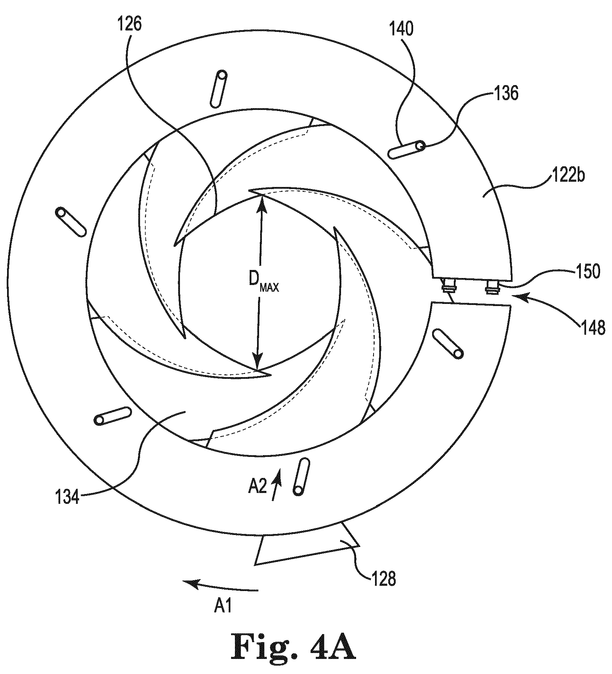

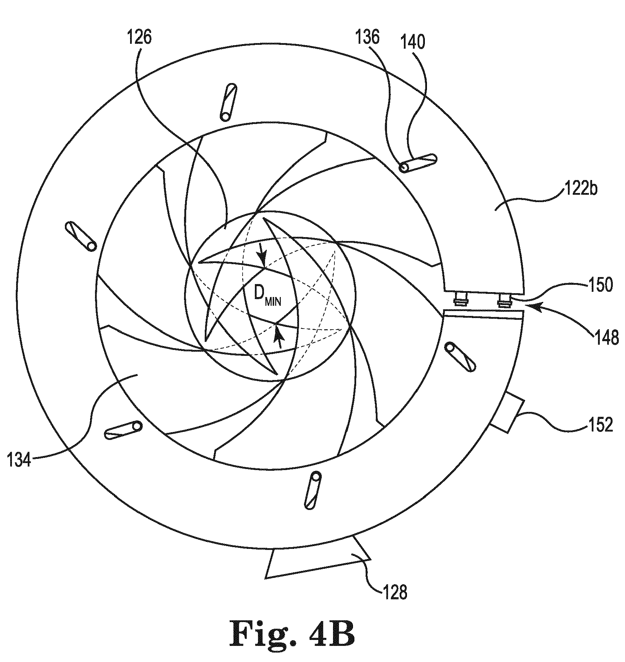

The movable element 28 is sized to be palpated through the perineal skin PS (Figure 1) in order for the patient to adjust the diameter D of the iris diaphragm 26. For example, the movable element 28 is sized so that the patient can palpate and engage the movable element 28 through the perineal skin surface using one or more fingers, e.g. the thumb and index finger of one hand. Moving the movable element 28 changes the diameter D of the iris diaphragm 26. Moving the movable element 28 in a first direction will result in a reduction of the diameter D of the iris diaphragm 26, while moving the movable element in a second, opposite direction will result in an increase of the diameter D. In a first, coapting position, the iris diaphragm 26 has a diameter Dmin and in a second, open position the iris diaphragm 26 has a diameter Dmax (Figures 4A & 4B).

The iris diaphragm 26 includes a number of fins 34 each configured to stack at least partly on a neighboring fin in a layered relationship which allows the fins 34 to slide on top of each other. In one embodiment shown in Figure 2, the iris diaphragm 26 has a number of twelve fins 34, but in other embodiments the iris diaphragm 26 includes from two to more than twelve fins 34. In one embodiment, the fins 34 generally have a shape comparable to a shark fin and having curved and/or rounded edges. In one embodiment, one end of a fin 34 includes a pointy top portion and another end includes a curved base edge having a width extending between two points. In one embodiment, curved edges extend from the two base edge points to the pointy top portion. The curved edges, the curved base edge and the pointy top portion span a plane between them defining first and second major surfaces of the fin 34 on each opposite side of the plane.

In one embodiment, movable element 28 is provided monolithically with the casing 22. Movement of the movable element 28 will move the casing 22 a distance equal to the movement of the movable element 28 in either the first or the second direction. In one embodiment, the casing 22 includes a monolithic casing provided with an internal track or channel in an interior surface of the casing 22. The track or channel provides support for and space for movement of the iris diaphragm 26.

Figure 3 is a perspective exploded view of one embodiment of an AUS 120 wherein casing 122 includes generally annular first and second casing members 122a, 122b. The first and second casing members 122a, 122b are adapted to accommodate iris diaphragm 126 in a layered configuration with the iris diaphragm 126 located between the first member 122a and the second member 122b. In one embodiment, the first and second casing members 122a, 122b are provided as washer-like elements. The first and second casing members 122a, 122b include a closable gap in their periphery to assist in positioning the AUS 120 around the urethra during surgery.

In one embodiment, the iris diaphragm 126 includes six individual fins 134. Each of the fins 134 is connected to at least one of the first and second members 122a, 122b. In one embodiment, each of the fins 134 includes a pin 136 extending orthogonally away from a first major surface 138a of the fin 134. The second casing member 122b includes a slot 140 to receive and provide a track for pin 136 to slide in. Second casing member 122b includes a number of slots 140 corresponding to the number of fins 134 having a pin 136. In the embodiment of Figure 3, the slots 140 are illustrated as extending all the way through a thickness of the second casing member 122b from interior surface 130b to exterior surface 132b. In one embodiment, the slots 140 extend only part-way through the thickness of second casing element 122b. In one embodiment, a pin 136 is provided also on a second major surface 138b of the fin 134.

Each individual fin 134 includes hole 142 to receive a protrusion 144 provided on interior surface 130a of first casing member 122a. The hole 142 and the protrusion 144 combine to provide a pivot link between each individual fin 134 and the first casing member 122a. The hole 142 does not necessarily have to extend all the way through a thickness of the fin 134 between its first and second major surfaces 138a, 138b.

The AUS 120 further includes movable element 128 provided on an outer periphery of, and monolithically with, the second casing member 122b. When the first and second casing members 122a, 122b and the iris diaphragm 126 are combined in the layered configuration of the AUS 120, movement of the movable element 128 in a first direction causes the pins 136 to slide in the slots 140 and the fins 134 to pivot around protrusions 144, which reduces the diameter D of the iris diaphragm 126. Moving the movable element 128 in the second, opposite direction causes the pins 136 to reverse in slots 140 and fins 134 to pivot oppositely around protrusions 144, which increases the diameter D of the iris diaphragm 126. In one embodiment, the first and the second casing members 122a, 122b are attachable to each other.

In one embodiment shown in Figure 3, the casing 122 of AUS 120 has an open configuration with each of the first and the second casing members 122a, 122b including a gap 146a, 146b. At least one of the first and second casing members 122a, 122b includes a lock 148. Engaging lock 148 results in the casing 122 being maintained in a closed configuration.

Figure 4A is a top view of one embodiment of the AUS 120 illustrated in Figure 3. Figure 4A shows the second casing member 122b and six fins 134 of the iris diaphragm 126 in a second, open position wherein the iris diaphragm 126 is adjusted to an open diameter Dmax. The first casing member 122a is not visible in Figure 4A. Pin 136 of each of the fins 134 of the iris diaphragm 126 is received in corresponding slot 140 of the second casing member 122b. By moving the movable element 128 in the direction of arrow A1, each pin 136 slides in its respective slot 140 in the direction of arrow A2 towards an inner periphery of the second casing member 122b. Each respective fin 134 moves by sliding on a neighboring fin to a first, coapting position wherein the diameter Dmin of the iris diaphragm 126 is reduced compared to the diameter Dmax in the second, open position.

Figure 4B is another top view of one embodiment of AUS 120 illustrated in Figure 3 showing the first, coapting position of the iris diaphragm 126 in which it is adjusted to a closed diameter Dmin. The length of the slots 140 define the distance the pins 136 can travel and therefore contributes to controlling the dimensions of the closed diameter Dmin and the open diameter Dmax of the iris diaphragm 126. Diameter Dmin of the iris diaphragm 126 is configured to be small enough to provide a sufficient coapting pressure on the outer surface of the urethra without damaging the urethra's surface and/or impeding necessary blood supply to the tissue. Diameter Dmax of the iris diaphragm 126 is configured to be large enough to allow unhindered flow of urine through the urethra.

In one embodiment, second casing member 122b includes a lock 148. Lock 148 provides for second casing member 122b to be maintained in a closed configuration. In one embodiment, the lock 148 includes two knobs 150 sized to engage with corresponding recesses (not shown) in the second casing member 122b in order to place the casing in its closed configuration. In one embodiment, the casing has an annular shape in the closed configuration. Other numbers of knobs and recesses for lock 148 are acceptable. A similar lock 148 can be included in first casing member 122a. Other types of locks are acceptable to maintain the casing in the closed configuration.

In one embodiment, a fixed member 154 is associated with casing 122, in Figure 4B shown as located at the second casing member 122b. The fixed member 154 provides a finger hold that is also palpable through the perineal skin. The finger hold is useful as a support for one or more fingers while another finger engages the movable element 128 to move it.

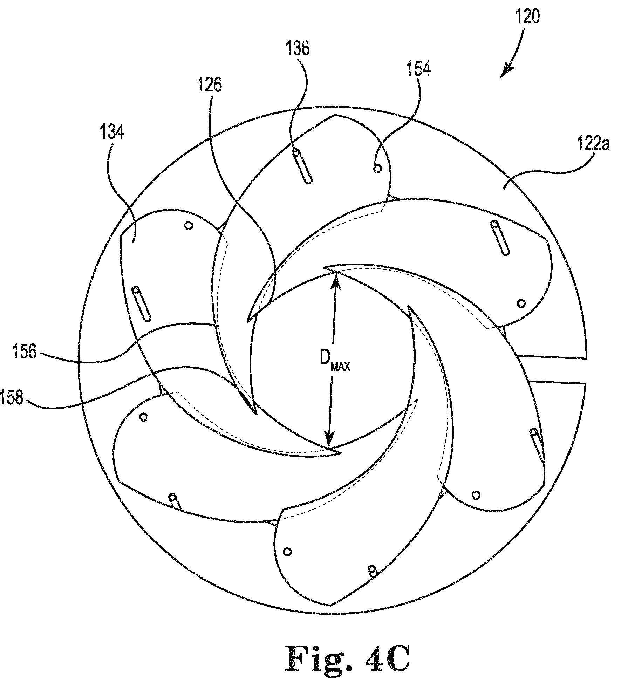

Figure 4C is another top view of one embodiment of an AUS 120. In this view, the second casing member 122b is left out to illustrate the fins 134 of the iris diaphragm 126 placed superior to first casing member 122a. Hole 142 of each fin 134 is placed on protrusion 144 (Figure 3) to provide a pivot point 154 for each individual fin 134 to rotate about in use. In one embodiment, the coaptation valve is permanently fixed to the casing 122. In one embodiment, the permanent fixing is provided through the connection between hole 142 of fin 134 and protrusion 144 extending from first casing member 122a.

In one embodiment as schematically indicated by the transparency of each fin 134 in Figure 4C, a portion 156 of each of the fins 134 is provided in a stacked relationship with a neighboring fin. In the embodiment of the AUS 120 of Figure 4C, the iris diaphragm 126 is shown in its second, open position with diameter Dmax. In this position, portion 156 includes a pointy top 158 of each fin 134. In the first, coapting position of the iris diaphragm 126 as illustrated in Figure 4B, portion 156 includes a larger part of each fin 134 stacked on a neighboring fin. In one embodiment, each fin 134 may overlap more than just the immediate neighboring fin, however not necessarily in stacking relationship with other than the immediate neighboring fin. When the movable element 128 is moved to change the diameter of the iris diaphragm 126, stacked fins 134 slide on each other in a mutually supporting manner.

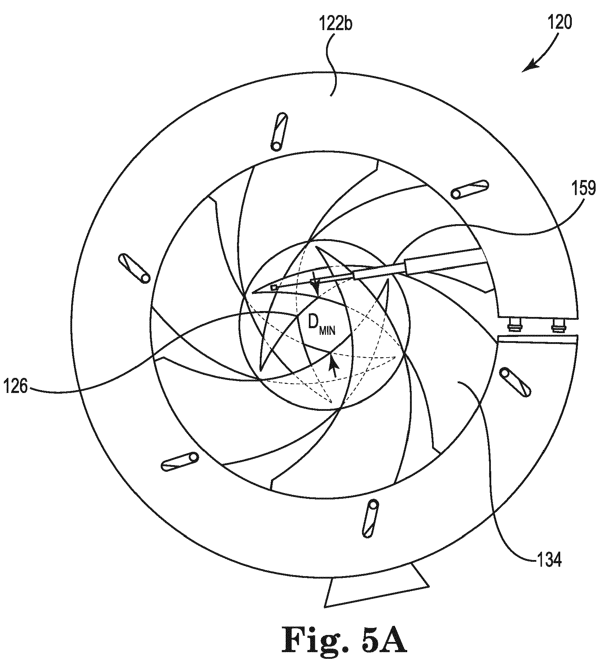

Figure 5A is a top view of the AUS 120 including a biasing member 159 adapted to maintain the iris diaphragm 126 in a first, coapting position in which the iris diaphragm 126 is adjusted to the diameter Dmin. In one embodiment, the biasing member 159 is connected to one of the fins 134 and to the second casing member 122b. In one embodiment, the biasing member 159 is pivotally connected to the second casing member 122b.

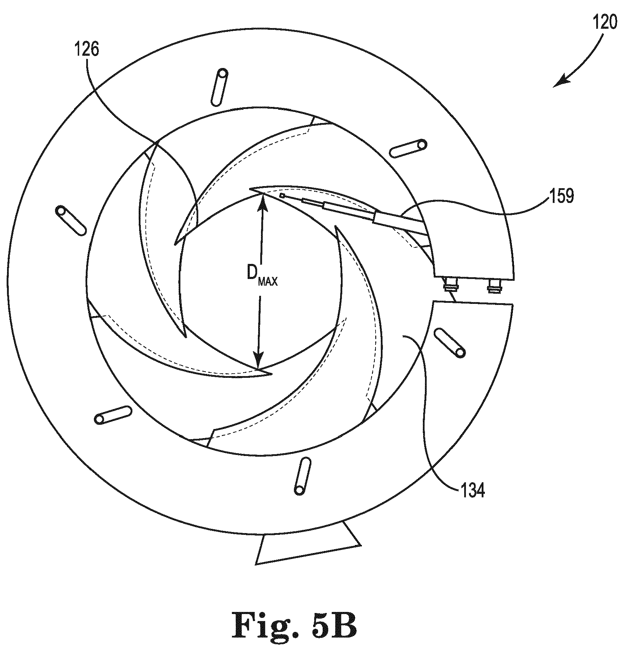

Figure 5B is a top view similar to Figure 5A showing embodiment situation in which the iris diaphragm 126 is adjusted to its second, open position with diameter Dmax and the biasing member 159 is in an engaged configuration corresponding to a situation where the patient wishes to void his bladder.

In one embodiment, the biasing member 159 is provided as a telescopic rod adapted to maintain the iris diaphragm in the first, coapting position. When the AUS 120 is in a continence-providing state the biasing member 159 bias the iris diaphragm to the closed diameter Dmin. When the patient wants to void his bladder, he palpates the movable element 128 through the perineal skin and moves the movable element 128 in a first direction to open the iris diaphragm 126 and thereby the biasing member 159 adapts to the engaged configuration of Figure 5B.

The biasing member 159 is adapted to move the iris diaphragm 126 to the first, coapting position from the second, open position of the iris diaphragm 126 without palpating the movable element 128 through the perineal skin. Put differently, the biasing member 159 has sufficient bias to move automatically (without any adjustment needed by the patient) from the engaged configuration exemplified in Figure 5B to the continence-providing state illustrated in Figure 5A. In one embodiment, the biasing member 159 is configured to impede the movement of the iris diaphragm 126 such that it takes the iris diaphragm 126 approximately between 45-180 seconds to reach the first, coapting position from the second, open position of the iris diaphragm 126. This is advantageous in that the AUS 120 reduces the required interaction by the patient to merely moving the movable element 128 to the second, open position of the iris diaphragm 126. When the bladder has been voided, the patient can go back to what he was doing before voiding since it is not required for the patient to actively move the movable element 128 back to the first, coapting position of the iris diaphragm 126 as the biasing member 159 ensures that this happens automatically. Depending on a timing of the patient's average voiding time and pattern, the biasing member 159 can be chosen or adjusted before surgery to match an appropriate time for returning to the first, coapting position of the iris diaphragm 126. However, the AUS 120 allows for the patient to manually return to the first, coapting position.

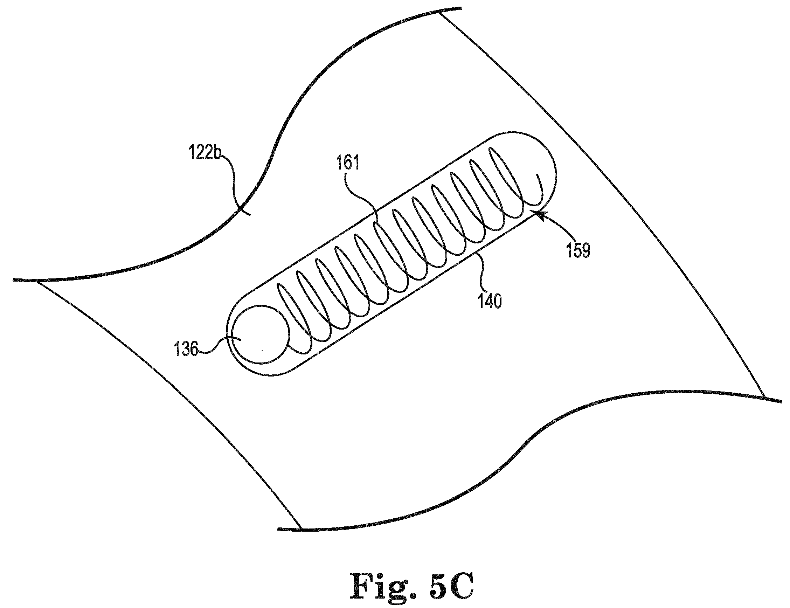

Figure 5C in an enlarged top view of another embodiment of a biasing member 159 provided as a coiled spring 161 sized to be located in slot 140 of the second casing member 122b. The coiled spring 161 attaches to second casing member 122b at one end and engages pin 136 at the opposite end of slot 140. In the continence providing configuration of the AUS 120, the coiled spring 161 extends throughout slot 140 and maintains the iris diaphragm 126 in the first, coapting position. When the patient wants to void the bladder, movement of the movable element 128 causes spring 161 to compress in an engaged configuration near the end of slot 140 where it is attached to second casing member 122b. In one embodiment, the coiled spring 161 includes a retaining member (not shown). In one embodiment, the retaining member includes a ratchet along its sides (not shown) to engage with pin 136 to delay the return of the spring 161 to its extended state thereby impeding the movement of the iris diaphragm 126. Other types of biasing and retaining members are acceptable. More than one biasing and/or retaining member can be included in the AUS.

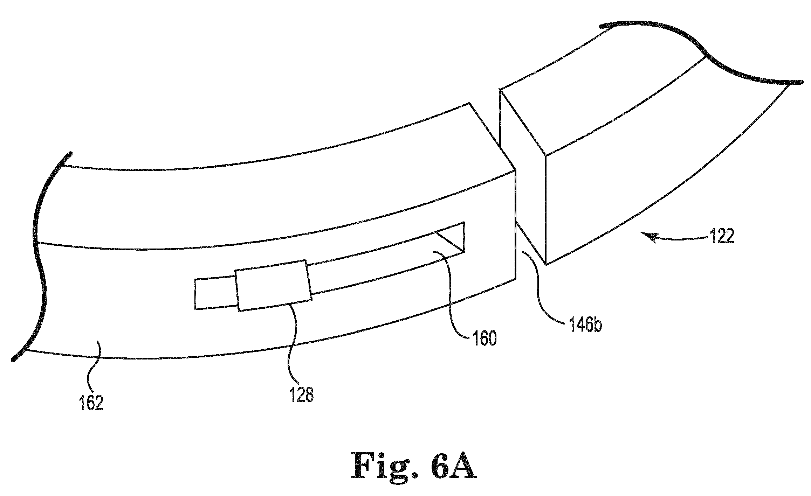

Figure 6A is an enlarged perspective view of a portion of casing 122 wherein a slit 160 is provided in the casing to accommodate movable element 128. In one embodiment, the slit 160 is provided such that movable element 128 is slidable along an external peripheral surface 162 of the casing 122. In one embodiment, the slit 160 is provided in the casing 122 near gap 146b. In one embodiment, the movable element 128 is configured as an individual part associated with the casing 122 and connected to iris diaphragm 126 via a direct link (not shown).

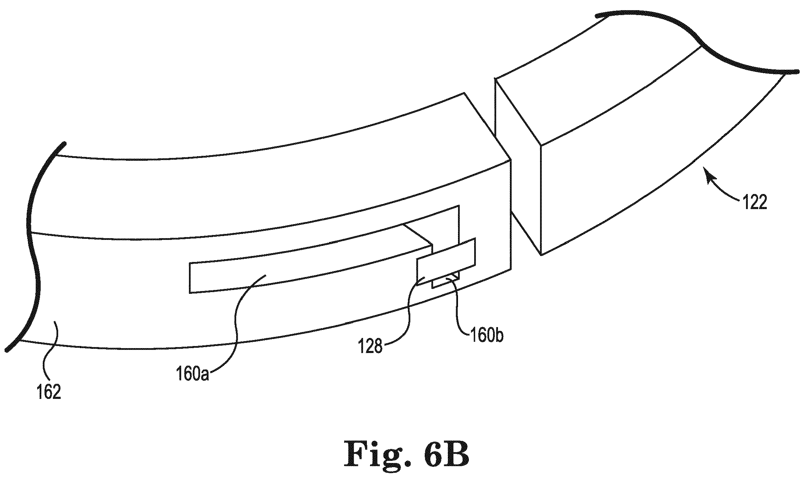

Figure 6B is an enlarged perspective view similar to Figure 6A showing a portion of casing 122 wherein a slit 160 is provided in the casing to accommodate movable element 128. In one embodiment the slit 160 includes at least a first section 160a and a second section 160b provided at an angle to the first section 160a. The angle is configured to be 90 degrees or more, i.e. forming an acute angle between the sections 160a, 160b. In one embodiment, the second section 160b of the slit provides a hold position for movable element 128, in which hold position the movable element 128 is secured against movement. In one embodiment, the hold position of the movable element 128 corresponds to the second, open position of the iris diaphragm 126. Placing the movable element 128 in the hold position thus maintains the iris diaphragm 126 open for voiding of the bladder for as long as required by the patient. In one embodiment, the iris diaphragm 126 moves away from engagement with the urethra when the movable element 128 is positioned in the second section 160b of the slit 160.

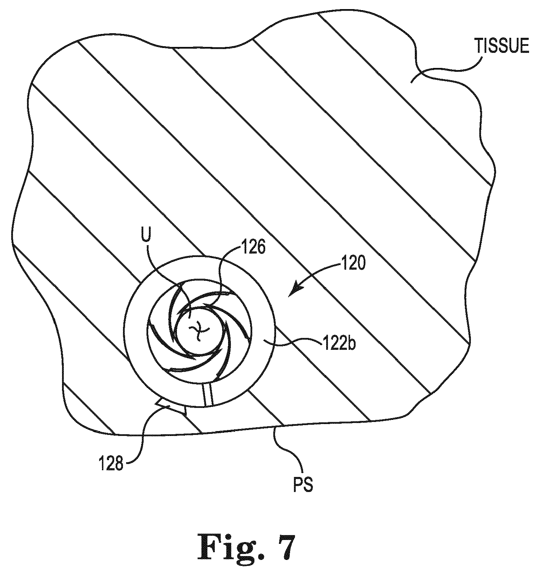

Figure 7 is a schematic view of the AUS 120 implanted in tissue in the perineal region of a patient and located around bulbar urethra U. The AUS 120 is shown with the iris diaphragm 126 adjusted to a first, coapting position. The AUS 120 is implanted such that the movable element 128 is located close to the perineal skin surface PS of the patient. This location of the movable element 128 of the AUS 120 provides for the patient to palpate the movable member 128 through the perineal skin in order to adjust the diameter of the iris diaphragm 126.

In one embodiment, the casing 122 and/or the iris diaphragm 126 include a protective membrane. In one embodiment, the membrane includes a silicone or other biocompatible coating provided on the surfaces of the first and second casing members 122a, 122b and on each individual fin 134. In one embodiment, only portions of the casing and the iris diaphragm 126 include the coating. The coating is useful for securing the tissue against the presence of sharp edges and/or to ensure biocompatibility.

Suitable materials for fabricating the fins 134 of the iris diaphragm 126 and the casing 122 of the AUS 120 include polymer materials, such as, but not limited to polypropylene, polyurethane, silicone or combinations thereof. In embodiments including a protective membrane or coating, the fins 134 and the casing 122 include metal materials including titanium, nitinol, stainless steel or other metals. Combination of polymer and metal materials are also acceptable.

In an aspect, the application relates to a kit of parts for providing urinary continence.

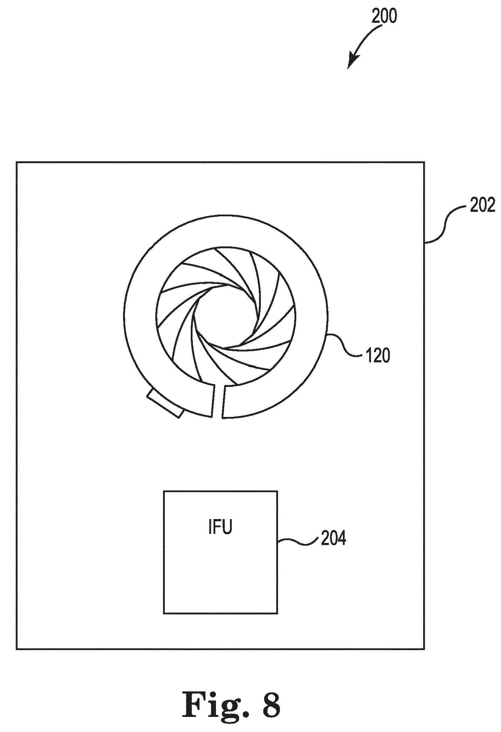

Figure 8 shows one embodiment wherein a kit of parts 200 includes a packaging 202, an AUS 120 and a set of instructions for use (IFU) 204. The IFU includes instructions and directions to guide the surgeon on how to use the kit of parts. The instructions include the following: initially, the packaging 202 is opened so that every element of the kit of parts is easily accessible to the surgeon. A desired location for placement of the AUS 120 is determined by the surgeon. The AUS 120 is put in position in the bulbous spongiosis muscle around the urethra. The surgeon will confirm that the diameter Dmin of the iris diaphragm 126 of the AUS 120 is not so small that blood supply to the urethral tissue is impeded or otherwise irritates the urethral tissue. The AUS 120 is positioned such that the movable element 128 of the AUS 120 is located near the perineal skin surface of the patient and that the movable element 128 is palpable and movable through the perineal skin. The IFU may additionally include instructions and directions on how to use the AUS 120 once it is implanted.

The application also discloses an exemplary method of providing urinary voiding control in a male patient.

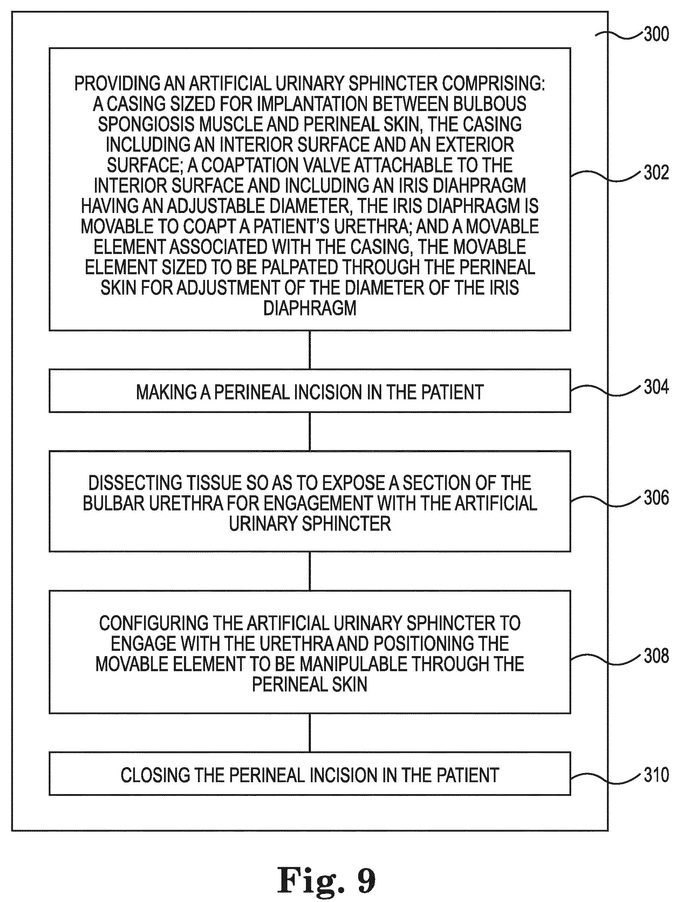

Figure 9 is a block diagram showing one example 300 of a method of providing urinary voiding control in a male patient. At 302 the method includes providing an AUS, for example AUS 20 or AUS 120 including a casing sized for implantation between bulbous spongiosis muscle and perineal skin and having an interior surface and an exterior surface; a coaptation valve attachable to the interior surface and including an iris diaphragm having an adjustable diameter, the iris diaphragm is movable to coapt a patient's urethra; and a movable element associated with the casing wherein the movable element is sized to be palpated through the perineal skin for adjustment of the diameter of the iris diaphragm. At 304 the method includes making a perineal incision in the patient. At 306 the method includes dissecting tissue so as to expose a section of the bulbar urethra for engagement with the AUS 20, 120. At 308 the method includes configuring the AUS 20, 120 to engagement with the urethra and positioning the movable element to be manipulable through the perineal skin. At 310 the method includes closing the perineal incision in the patient.

Thereby, the method provides for placing of the AUS 20, 120 around the bulbar urethra of a male patient to provide urinary voiding control in the patient. The implanted AUS 20, 120 has a continence-providing resting state in which the iris diaphragm has a diameter Dmin and coapts the urethra and an engaged state in which the iris diaphragm has a diameter Dmax allowing for urine flow through the urethra. The method thus provides the patient control of voiding of his bladder by implanting and engaging the AUS 20, 120 with the urethra.

The application also discloses an exemplary method of controlling urinary voiding in a male patient.

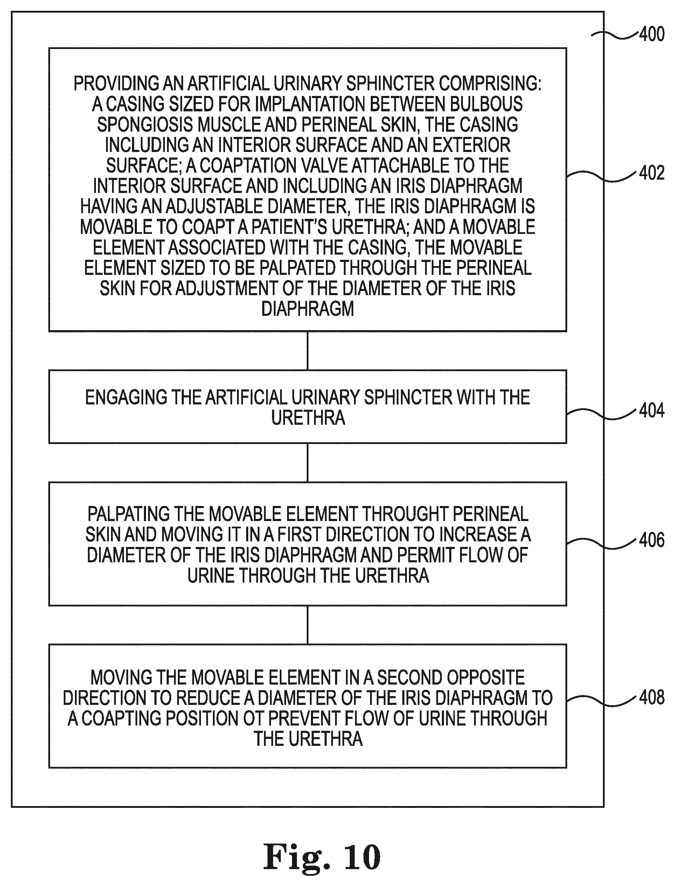

Figure 10 is a block diagram showing one example 400 of a method of controlling urinary voiding in a male patient. At 402 the method includes providing an AUS, for example AUS 20 or AUS 120 including a casing sized for implantation between bulbous spongiosis muscle and perineal skin and having an interior surface and an exterior surface; a coaptation valve attachable to the interior surface and including an iris diaphragm having an adjustable diameter, the iris diaphragm is movable to coapt a patient's urethra; and a movable element associated with the casing wherein the movable element is sized to be palpated through the perineal skin for adjustment of the diameter of the iris diaphragm. At 404 the method includes engaging the AUS 20, 120 with the urethra. At 406 the method includes palpating the movable element through perineal skin and moving it in a first direction to increase a diameter of the iris diaphragm and permit flow of urine through the urethra. At 408 the method includes moving the movable element in a second, opposite direction to reduce a diameter of the iris diaphragm to a coapting position to prevent flow of urine through the urethra. In one example, moving the movable element in the second, opposite direction includes palpating and manipulating the movable member through the perineal skin.

Thereby, the exemplary method provides for controlling of urinary voiding in a male patient. The diameter of the iris diaphragm can be changed at the will of the patient to shift the AUS 20, 120 between an open position and a coapting position and thus provides urinary continence in the patient. The AUS 20, 120 is shifted to the coapting position without requiring the patient to perform the shifting.

An artificial urinary sphincter has been presented that is less invasive in nature since the AUS itself is the only part to be implanted. The AUS it furthermore easily controlled by the patient himself by moving the movable element through the perineal skin.

Although specific embodiments have been illustrated and described herein, it will be appreciated by those of ordinary skill in the art that a variety of alternate and/or equivalent implementations may be substituted for the specific embodiments shown and described without departing from the scope of the present invention. This application is intended to cover any adaptations or variations of medical devices as discussed herein. Therefore, it is intended that this invention be limited only by the claims and the equivalents thereof.

## Claims

1. An artificial urinary sphincter (20) comprising:
a casing (22) sized for implantation between bulbous spongiosis muscle and perineal skin, the casing (22) including an interior surface (130) and an exterior surface (32);
a coaptation valve (24) attachable to the interior surface (130) and including an iris diaphragm (26) having an adjustable diameter, the iris diaphragm (26) is movable to coapt a patient's urethra;
a movable element (28) associated with the casing (22),
wherein the movable element (28) is sized to be palpated through the perineal skin for adjustment of the diameter of the iris diaphragm (26);
a biasing member (159) connected to the iris diaphragm (26) and to the casing (22) and adapted to maintain the iris diaphragm (26) in a first, coapting position;
**characterized in that** the biasing member (159) is adapted to move the iris diaphragm (26) to the first, coapting position from a second, open position of the iris diaphragm (26) without manual adjustment.

2. The artificial urinary sphincter of claim 1, wherein the biasing member (159) is configured to impede the movement of the iris diaphragm (26) such that it takes the iris diaphragm (26) approximately between 45-180 seconds to reach the first, coapting position from the second, open position of the iris diaphragm (26).

3. The artificial urinary sphincter of claim 1, wherein the coaptation valve (24) is fixedly attached to the casing (22).

4. The artificial urinary sphincter of claim 1, wherein the casing (22) is adapted to have an open and a closed configuration.

5. The artificial urinary sphincter of claim 4, wherein the casing (22) has an overall annular shape in the closed configuration.

6. The artificial urinary sphincter of claim 4, wherein the casing (22) comprises a lock (148) to maintain the casing (22) in the closed configuration.

7. The artificial urinary sphincter of claim 1, wherein the movable element (28) is accommodated in a slit (160) provided in the casing (22).

8. The artificial urinary sphincter of claim 7, wherein the slit (160) comprises at least a first (160a) and a second section (160b) provided at an angle to each other.

9. The artificial urinary sphincter of claim 8, wherein the second section (160b) of the slit (160) provides a hold position for the movable element (28) in which hold position the movable element (28) is secured against movement.

10. The artificial urinary sphincter of claim 1, further comprising a fixed member (154) associated with the casing (22), the fixed member (154) providing a finger hold.

11. The artificial urinary sphincter of claim 1, wherein the movable element (28) is directly linked to the iris diaphragm (26).

12. The artificial urinary sphincter of claim 1, wherein the casing (22) comprises a generally annular first member (122a) and a generally annular second member (122b) adapted to accommodate the iris diaphragm (26) in a layered configuration with the iris diaphragm (26) located between the first member (122a) and the second member (122b) and the iris diaphragm (26) connected to at least one of the first and second members (122a, 122b).

13. The artificial urinary sphincter of claim 12, wherein the first and second members (122a, 122b) of the casing (22) are attachable to each other.

14. The artificial urinary sphincter of claim 1, wherein the iris diaphragm (26) comprises between 2 and 12 fins (34, 134).

15. The artificial urinary sphincter of claim 14, wherein the iris diaphragm (26) comprises 6 fins (34, 134).

16. The artificial urinary sphincter of claim 14, wherein each fin (34, 134) is provided in a stacked relationship with a neighboring fin (34, 134).

17. The artificial urinary sphincter of claim 1, wherein the casing (22) and/or the iris diaphragm (26) comprises a protective membrane.

18. A kit of parts (200) for providing urinary continence, comprising:
a packaging (202);
an artificial urinary sphincter (120) according to claim 1; and
a set of instructions for use (204) of the artificial urinary sphincter (120).

## Patentansprüche

1. Künstlicher Blasenschließmuskel (20), umfassend:
ein Gehäuse (22) mit einer Größe zur Implantation zwischen Musculus bulbospongiosus und Perineum, wobei das Gehäuse (22) eine Innenfläche (130) und eine Außenfläche (32) aufweist;
ein Koaptationsventil (24), das an der Innenfläche (130) befestigt werden kann und ein Irisdiaphragma (26) mit einem verstellbaren Durchmesser aufweist, wobei das Irisdiaphragma (26) zur Koaptation der Harnröhre des Patienten beweglich ist;
ein bewegliches Element (28), das mit dem Gehäuse (22) in Verbindung steht,
wobei das bewegliche Element (28) eine Größe zur Palpation durch das Perineum zur Anpassung des Durchmessers des Irisdiagphragmas (26) aufweist;
ein Vorspannelement (159), das mit dem Irisdiaphragma (26) und dem Gehäuse (22) verbunden ist und ausgelegt ist, das Irisdiaphragma (26) in einer ersten Koaptationsstellung zu halten;
**dadurch gekennzeichnet, dass** das Vorspannelement (159) ausgelegt ist, das Irisdiaphragma (26) in die erste Koaptationsstellung aus einer zweiten offenen Stellung des Irisdiaphragmas (26) ohne manuelle Verstellung zu bewegen.

2. Künstlicher Blasenschließmuskel nach Anspruch 1, wobei das Vorspannelement (159) ausgelegt ist, die Bewegung des Irisdiaphragmas (26) zu behindern, so dass das Irisdiaphragma (26) ungefähr 45-180 Sekunden benötigt, um die erste Koaptationsstellung aus der zweiten offenen Stellung des Irisdiaphragmas (26) zu erreichen.

3. Künstlicher Blasenschließmuskel nach Anspruch 1, wobei das Koaptationsventil (24) fest an dem Gehäuse (22) befestigt ist.

4. Künstlicher Blasenschließmuskel nach Anspruch 1, wobei das Gehäuse (22) ausgelegt ist, eine offene und eine geschlossene Konfiguration aufzuweisen.

5. Künstlicher Blasenschließmuskel nach Anspruch 4, wobei das Gehäuse (22) eine insgesamt ringförmige Gestalt in der geschlossenen Konfiguration aufweist.

6. Künstlicher Blasenschließmuskel nach Anspruch 4, wobei das Gehäuse (22) eine Verschlussvorrichtung (148) umfasst, um das Gehäuse (22) in der geschlossenen Konfiguration zu halten.

7. Künstlicher Blasenschließmuskel nach Anspruch 1, wobei das bewegliche Element (28) in einem im Gehäuse (22) bereitgestellten Schlitz (160) untergebracht ist.

8. Künstlicher Blasenschließmuskel nach Anspruch 7, wobei der Schlitz (160) wenigstens einen ersten (160a) und einen zweiten (160b) Abschnitt umfasst, die in einem Winkel zueinander bereitgestellt sind.

9. Künstlicher Blasenschließmuskel nach Anspruch 8, wobei der zweite Abschnitt (160b) des Schlitzes (160) eine Haltestellung für das bewegliche Element (28) bereitstellt, wobei das bewegliche Element (28) in dieser Haltestellung gegen eine Bewegung gesichert ist.

10. Künstlicher Blasenschließmuskel nach Anspruch 1, ferner umfassend ein fixiertes Element (154), das mit dem Gehäuse (22) in Verbindung steht, wobei das fixierte Element (154) eine Fingerhalterung bereitstellt.

11. Künstlicher Blasenschließmuskel nach Anspruch 1, wobei das bewegliche Element (28) direkt mit dem Irisdiaphragma (26) verbunden ist.

12. Künstlicher Blasenschließmuskel nach Anspruch 1, wobei das Gehäuse (22) ein allgemein ringförmiges erstes Element (122a) und ein allgemein ringförmiges zweites Element (122b) umfasst, die ausgelegt sind, das Irisdiaphragma (26) in einer Schichtkonfiguration aufzunehmen, wobei das Irisdiaphragma (26) zwischen dem ersten Element (122a) und dem zweiten Element (122b) angeordnet ist und das Irisdiaphragma (26) mit wenigstens einem der ersten und zweiten Elemente (122a, 122b) verbunden ist.

13. Künstlicher Blasenschließmuskel nach Anspruch 12, wobei die ersten und zweiten Elemente (122a, 122b) des Gehäuses (22) aneinander befestigt werden können.

14. Künstlicher Blasenschließmuskel nach Anspruch 1, wobei das Irisdiaphragma (26) zwischen 2 und 12 Finnen (34, 134) umfasst.

15. Künstlicher Blasenschließmuskel nach Anspruch 14, wobei das Irisdiaphragma (26) 6 Finnen (34, 134) umfasst.

16. Künstlicher Blasenschließmuskel nach Anspruch 14, wobei jede Finne (34, 134) in einer gestapelten Beziehung mit einer benachbarten Finne (34, 134) bereitgestellt ist.

17. Künstlicher Blasenschließmuskel nach Anspruch 1, wobei das Gehäuse (22) und/oder das Irisdiaphragma (26) eine Schutzmembran umfasst.

18. Teilesatz (200) zur Bereitstellung von Harnkontinenz, umfassend:
eine Verpackung (202);
einen künstlichen Blasenschließmuskel (120) nach Anspruch 1; und
eine Gebrauchsanweisung (204) für den künstlichen Blasenschließmuskel (120).

## Revendications

1. Sphincter urinaire (20) artificiel comprenant :
un boîtier (22) dimensionné pour être implanté entre muscle spongieux bulbeux et peau périnéale, le boîtier (22) comprenant une surface intérieure (130) et une surface extérieure (32) ;
une valve de coaptation (24) pouvant être fixée à la surface intérieure (130) et comprenant un diaphragme à iris (26) ayant un diamètre réglable, le diaphragme à iris (26) étant mobile pour coapter l'urètre d'un patient ;
un élément mobile (28) associé au boîtier (22), dans lequel l'élément mobile (28) est dimensionné pour être palpé à travers la peau périnéale en vue du réglage du diamètre du diaphragme à iris (26) ;
un élément de sollicitation (159) relié au diaphragme à iris (26) et au boîtier (22) et apte à maintenir le diaphragme à iris (26) dans une première position de coaptation,
**caractérisé en ce que** l'élément de sollicitation (159) est apte à déplacer le diaphragme à iris (26) vers la première position de coaptation à partir d'une seconde position d'ouverture du diaphragme à iris (26) sans réglage manuel.

2. Sphincter urinaire artificiel selon la revendication 1, dans lequel l'élément de sollicitation (159) est configuré pour entraver le mouvement du diaphragme à iris (26) de telle sorte qu'il faille environ entre 45 et 180 secondes au diaphragme à iris (26) pour atteindre la première position de coaptation à partir de la seconde position d'ouverture du diaphragme à iris (26).

3. Sphincter urinaire artificiel selon la revendication 1, dans lequel la valve de coaptation (24) est fixée à demeure au boîtier (22).

4. Sphincter urinaire artificiel selon la revendication 1, dans lequel le boîtier (22) est apte à présenter une configuration ouverte et une fermée.

5. Sphincter urinaire artificiel selon la revendication 4, dans lequel le boîtier (22) présente une forme globalement annulaire en configuration fermée.

6. Sphincter urinaire artificiel selon la revendication 4, dans lequel le boîtier (22) comprend un verrou (148) pour maintenir le boîtier (22) en configuration fermée.

7. Sphincter urinaire artificiel selon la revendication 1, dans lequel l'élément mobile (28) est logé dans une fente (160) disposée dans le boîtier (22).

8. Sphincter urinaire artificiel selon la revendication 7, dans lequel la fente (160) comprend au moins une première section (160a) et une seconde section (160b) disposées pour former un angle l'une avec l'autre.

9. Sphincter urinaire artificiel selon la revendication 8, dans lequel la seconde section (160b) de la fente (160) assure une position de maintien de l'élément mobile (28), position de maintien dans laquelle l'élément mobile (28) est immobilisé.

10. Sphincter urinaire artificiel selon la revendication 1, comprenant en outre un élément fixe (154) associé au boîtier (22), l'élément fixe (154) fournissant un maintien par doigt.

11. Sphincter urinaire artificiel selon la revendication 1, dans lequel l'élément mobile (28) est relié directement au diaphragme à iris (26).

12. Sphincter urinaire artificiel selon la revendication 1, dans lequel le boîtier (22) comprend un premier élément (122a) dans l'ensemble annulaire et un second élément (122b) dans l'ensemble annulaire aptes à loger le diaphragme à iris (26) dans une configuration en couches, le diaphragme à iris (26) étant situé entre le premier élément (122a) et le second élément (122b) et le diaphragme à iris (26) étant raccordé au premier et/ou au second élément (122a, 122b).

13. Sphincter urinaire artificiel selon la revendication 12, dans lequel les premier et second éléments (122a, 122b) du boîtier (22) peuvent être fixés l'un à l'autre.

14. Sphincter urinaire artificiel selon la revendication 1, dans lequel le diaphragme à iris (26) comprend entre 2 et 12 lamelles (34, 134).

15. Sphincter urinaire artificiel selon la revendication 14, dans lequel le diaphragme à iris (26) comprend 6 lamelles (34, 134).

16. Sphincter urinaire artificiel selon la revendication 14, dans lequel chaque lamelle (34, 134) est disposée en empilement par rapport à une lamelle (34, 134) voisine.

17. Sphincter urinaire artificiel selon la revendication 1, dans lequel le boîtier (22) et/ou le diaphragme à iris (26) comprend une membrane protectrice.

18. Ensemble (200) de pièces pour offrir une continence urinaire, comprenant :
un emballage (202) ;
un sphincter urinaire (120) artificiel selon la revendication 1 ; et
un jeu (204) de mode d'emploi du sphincter urinaire (120) artificiel.
